# FASCICULE DE BREVET EUROPEEN

(11) **EP 1 660 466 B1**
(45) Date de publication et mention de la délivrance du brevet: **09.01.2008**
(21) Numéro de dépôt: 04786315.4
(22) Date de dépôt: 17.08.2004
(51) Int. Cl.: C07D 263/44

(54) **PROCEDE D'OBTENTION DE N-CARBOXYANHYDRIDES D'ALPHA-AMINOACIDES A PROTECTION URETHANE**
VERFAHREN ZUR GEWINNUNG VON URETHANGESCHÜTZTEN N-CARBOXYANHYDRIDEN VON ALPHA-AMINOSÄUREN
METHOD OF OBTAINING URETHANE-PROTECTED N-CARBOXYANHYDRIDES OF ALPHA AMINO ACIDS

(30) Priorité: 22.08.2003 FR 0310101
(43) Date de publication de la demande: 31.05.2006
(73) Titulaire: ISOCHEM, 75004 Paris (FR)
(72) Inventeur: LHERMITTE, Hervé, F-75019 Paris (FR); GRIMA, Julien, F-91150 Etampes (FR); PARIS, Antoine, F-76000 Rouen (FR)
(74) Mandataire: Warcoin, Jacques
(86) Numéro de dépôt international: PCT/FR2004/002148
(87) Numéro de publication internationale: WO 2005/021517

(56) Documents cités:
- EP-A- 0 492 254
- FULLER W D ET AL: "Urethane-protected alpha-amino acid N-carboxyanhydrides and peptide synthesis" BIOPOLYMERS, vol. 40, no. 2, 1996, pages 183-205, XP002278328 cité dans la demande
- OKUMURA K ET AL: "Dehydrooligopeptides. XIV. Synthesis of 2-[(Z)-1-amino-1-alken- 1-yl]oxazole-4-carboxylic acid and the main common skeleton of thiostrepton peptide antibiotics, A10255G and J" BULLETIN OF THE CHEMICAL SOCIETY OF JAPAN, vol. 69, no. 8, août 1996 (1996-08), pages 2309-2316, XP002278329

## Description

La présente invention concerne un nouveau procédé de préparation de N-carboxyanhydrides d'alpha-aminoacides à protection uréthane. Le nouveau procédé permet la synthèse de N-carboxyanhydrides d'alpha-aminoacides à protection uréthane à partir de N-carboxyanhydrides d'alpha-aminoacides en présence d'une quantité catalytique de triéthylène-diamine, sans ajout de base de type amine tertiaire en quantités importantes.

Les N-carboxyanhydrides d'alpha-aminoacides (ci-après dénommés sous l'abréviation NCAs), éventuellement protégés, sont des agents d'acylation fréquemment utilisés pour la formation de polyalpha-aminoacides à haut poids moléculaire et pour la production de dipeptides. Les NCAs sont des composés très réactifs, qui ne forment pas, par réarrangement notamment, de produits secondaires indésirés et dont l'unique sous-produit de réaction est le dioxyde de carbone. Une fois que le NCA est mis à réagir avec une fonction amine libre d'un acide aminé, du dioxyde de carbone est immédiatement libéré et il se forme un dipeptide, contenant lui aussi une fonction amine libre. Cette amine va réagir avec le NCA pour former un tripeptide et ainsi de suite. Les NCAs peuvent ainsi être utilisés dans la formation de poly(alpha aminoacides) mais ils ne peuvent pas être utilisés facilement dans la synthèse séquentielle de polypeptides, les réactions secondaires multiples de condensation, telles qu'une oligomérisation, étant difficiles à contrôler.

Des N-carboxyanhydrides d'alpha-aminoacides substitués par des groupements uréthane ont été décrits dans la littérature, ils sont utilisés dans les synthèses peptidiques. Le substituant uréthane apporte un haut degré de protection et il permet de minimiser les réactions de polymérisation durant la réaction de couplage. Les NCAs à protection uréthane, ci-après abrégés UNCAs, présentent tous les avantages des NCAs non substitués sans les inconvénients de ces derniers.

Les UNCAs permettent une synthèse contrôlée de polypeptides sans qu'une pré-activation des groupements carboxyles ne soit nécessaire et sans que l'ajout d'additifs, tels que le N-hydroxybenzotriazole, ne soit nécessaire. Ainsi, la purification des peptides préparés en solution est facilitée, étant donné que le seul sous-produit de la réaction de synthèse peptidique est le dioxyde de carbone.

Les UNCAs sont également très utiles en tant que matières premières dans la synthèse d'hormones ou de médicaments anti-sida.

Les UNCAs, qui sont sous forme cristalline dans des conditions de températures et de pression ambiantes, sont stables sous les conditions standard de manipulation au laboratoire, au stockage, et sous les conditions de synthèse peptidique.

Les deux voies de synthèse principales des UNCAs à partir de NCAs sont les suivantes :
1) Les UNCAs peuvent être synthétisés par condensation d'un chloroformiate d'alkyle ou d'aralkyle, tel que le Fmoc-Cl (le chloroformiate de 9-fluorènylméthyloxycarbonyle) ou le chloroformiate de benzyle, avec un NCA en présence d'une quantité au moins stoechiométrique d'une amine tertiaire. Cette amine tertiaire, qui est classiquement la N-méthylmorpholine, permet de piéger l'acide chlorhydrique libéré. Le NCA est ainsi mis en solution dans un solvant inerte, tel que le THF, et refroidi. 1,1 à 1,3 équivalent de chloroformiate d'alkyle ou d'aralkyle est ajouté en une seule fois puis au moins 1,5 équivalent d'une amine tertiaire, la N-méthylmorpholine par exemple, est ajouté lentement. La suspension résultante est laissée reposer 1 à 2 heures, à une température comprise entre - 25 et - 5°C. Ensuite de l'acide chlorhydrique dissous dans du dioxane est ajouté doucement jusqu'à l'obtention de valeurs de pH d'environ 4-5. Le chlorhydrate de l'amine tertiaire ainsi formé est éliminé par filtration et le UNCA est concentré puis cristallisé.
   Toutes les étapes du procédé sont réalisées sous atmosphère inerte (N₂) et tous les solvants sont séchés sur tamis moléculaire 4 Å avant d'être utilisés (William D. Fuller et al, Urethane-protected-alpha-amino acid N-carboxyanhydrides and peptide synthesis, Biopolymers, 1996, 40,183-205).
   Cette voie de synthèse est peu appropriée pour préparer certains N-carboxyanhydrides d'alpha-aminoacides protégés, notamment ceux protégés par un radical t-butoxylcarbonyle, le chloroformiate de t-butyle étant très instable au dessus de -20°C ou en présence d'amines tertiaires.
2) Les UNCAs peuvent également être synthétisés par condensation d'un dicarbonate de dialkyle avec un NCA. Cette réaction libère une molécule d'alcool et une molécule de dioxyde de carbone. Cette synthèse doit impérativement être effectuée en présence d'une quantité élevée, au moins 50% molaire par rapport à la quantité molaire de NCA engagée, d'une aminé tertiaire telle que la N-méthylmorpholine associée à une quantité catalytique de DMAP (4-diméthylaminopyridine) ou une pyridine (William D. Fuller et al, Urethane-protected-alpha-amino acid N-carboxyanhydrides and peptide synthesis, Biopolymers, 1996, 40, 183-205). Cette voie de synthèse est particulièrement adaptée à la synthèse de N-carboxyanhydrides d'alpha-aminoacides protégés par un radical t-butoxylcarbonyle en utilisant le ditertiobutyldicarbonate, voir également Bull. Chem. Soc. Japan, 69 (1996), 2309-2316.
   La demande WO89/08643 décrit des N-carboxyanhydrides d'alpha-aminoacides et des N-thiocarboxyanhydrides d'alpha-aminoacides à protection uréthane, de formule, 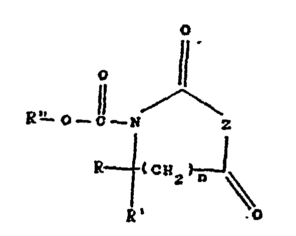
dans laquelle R et R' représentent un atome d'hydrogène, un radical alkyle, cycloalkyle, cycloalkyle substitué par un radical alkyle substitué, aryle ou aryle substitué et au moins un groupement R ou R' ne représente pas un atome d'hydrogène ; R" représente un radical alkyle, aryle, alkyle substitué ou aryle substitué ; Z représente un atome d'oxygène ou de soufre et n vaut 0, 1 ou 2.

Ces composés sont préparés par réaction d'un NCA avec un halogénoformate dans un solvant inerte, tel que le toluène, dans des conditions anhydre, en présence d'une base de type amine tertiaire ajoutée en excès.

Les procédés existants de synthèse de UNCAs ne sont pas satisfaisants. En effet, le meilleur procédé décrit ci-dessus, qui utilise une base de type amine tertiaire en quantité au moins égale à 50 % molaire par rapport à la quantité de NCA engagée, donne des rendements seulement d'environ 60 % à condition de sécher les solvants au tamis moléculaire 4 Å et d'opérer entre - 20 et -15 °C.

Il a été découvert que, de façon surprenante, l'utilisation de triéthylène-diamine (TEDA) en quantité catalytique très faible, inférieure à 5% molaire par rapport à la quantité molaire de NCA engagée, sans ajout de base de type amine tertiaire, conduit à d'excellents résultats.

Dans le cadre de la présente invention, l'abréviation NCA(s) désigne le(s) N-carboxyanhydride(s) d'alpha-aminoacide(s) et UNCA(s) désigne le(s) N-carboxyanhydride(s) d'alpha-aminoacide(s) à protection uréthane.
Au sens de la présente invention, on entend par quantité catalytique, une quantité nettement inférieure et plus précisément inférieure à 50% à celle requise par la stoechiométrie.

La présente invention concerne un procédé d'obtention de N-carboxyanhydrides d'alpha-aminoacides à protection uréthane (UNCAs), de formule I 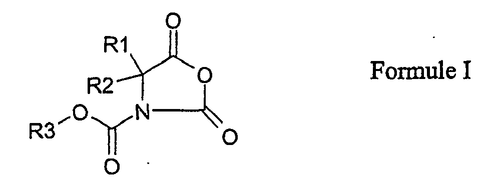 dans laquelle R1 et R2, identiques ou différents, représentent ensemble ou indépendamment l'un de l'autre un atome d'hydrogène ou une chaîne latérale d'un alpha-aminoacide naturel ou synthétique éventuellement porteuse de groupements fonctionnels le cas échéant protégés ; R³ représente un radical alkyle linéaire ou ramifié saturé ou insaturé en C₁-C₁₀ ou un radical aralkyle ou alkaryle de 7 à 14 atomes de carbone, caractérisé en ce qu'un N-carboxyanhydride d'alpha-aminoacide (NCA) de formule II, 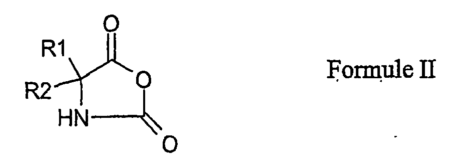 dans laquelle R1 et R2 ont la même signification que pour la formule I, est mis à réagir avec au moins un équivalent, par rapport à la quantité molaire engagée de NCA de formule II, de dicarbonate de formule III 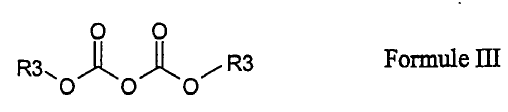 dans laquelle R3 a la même signification que pour la formule I, en présence d'une quantité catalytique de 1 ,4-diazabicylo[2.2.2]octane, également dénommé triéthylène-diamine (TEDA), par rapport à la quantité molaire engagée de NCA de formule II, dans un solvant organique inerte de point de fusion inférieur à environ -20 °C.

Un alpha-aminoacide naturel ou synthétique est acide aminé porteur sur le premier carbone de la chaîne d'une fonction amine et d'une fonction acide carboxylique. Le reste de l'alpha-aminoacide est appelé chaîne latérale de l'alpha-aminoacide.

R1 et R2 sont le cas échéant protégés par des groupements protecteurs habituellement utilisés dans le domaine des aminoacides et des peptides (Bodansky, Principles of peptide synthesis, Springer-Verlag, 1984; Alpha amino-acids N-carboxyanhydrides and related heterocycles, Hans R. Kricheldorf Springer Verlag, 1987).

R1 et R2, identiques ou différents, représentent avantageusement un atome d'hydrogène, un radical alkyle en C₁-C₈ linéaire ou ramifié comprenant éventuellement un ou plusieurs substituant(s) habituels dans le domaine des aminoacides et des peptides. Les substituants sont notamment choisis dans le groupe constitué par OH, SH, NH₂, NHC(NH)NH₂, CONH₂, O-alkyle en C₁-C₆, O-aryle en C₆-C₁₀, S-alkyle en C₁-C₆, COO-alkyle en C₁-C₆, COO-aralkyle en C₅-C₈, notamment le radical ester benzylique.

Un groupement R1 ou R2 peut avantageusement représenter un radical cycloalkyle en C₅-C₇ éventuellement substitué par un ou plusieurs groupement(s) habituel(s) dans le domaine des aminoacides et des peptides. Les substituants sont notamment choisis dans le groupe constitué par les halogènes, OH, O-alkyle en C₁-C₆, O-aryle en C₆-C₁₀, alkyle en C₁-C₆.

Un groupement R1 ou R2 peut avantageusement représenter un radical phényle, naphtyle, hétéroaromatique à 5 ou 6 chaînons ou indole éventuellement substitué par un ou plusieurs groupement(s) habituels dans le domaine des aminoacides et des peptides. Les substituants sont notamment choisis dans le groupe constitué par les halogènes, OH, O-alkyle en C₁-C₆, O-aryle en C₆-C₁₀, alkyle en C₁-C₆.

Pour des raisons évidentes d'encombrements stériques, R1 et R2 ne peuvent pas représenter simultanément un radical cyclique. Par radical cyclique, on entend ledit radical cycloalkyle en C₅-C₇ ainsi que ledit radical phényle, naphtyle, hétéroaromatique à 5 ou 6 chaînons ou indole.

R1 et R2 peuvent également former ensemble un radical cycloalkyle en C₅-C₇ éventuellement substitué par un ou plusieurs groupement(s) habituels dans le domaine des aminoacides et des peptides. Les groupements sont notamment choisis dans le groupe constitué par les halogènes, OH, O-alkyle en C₁-C₆, alkyle en C₁-C₆, O-aryle en C₆-C₁₀.

Dans le cas où R1 et R2 ne forment pas ensemble un radical cycloalkyle en C₅-C₇, avantageusement au moins un des groupements R1 et R2, tels que définis précédemment, représente un atome d'hydrogène.

Dans les composés de formule II, les groupements fonctionnels sont avantageusement protégés par des groupes protecteurs adaptés.

Selon une variante avantageuse de l'invention, R3 représente le radical méthyle, le radical éthyle, le radical tertio-butyle, le radical benzyle, le radical allyle ou le radical 9-fluorénylméthyle. En effet, bien qu'il existe une grande variété d'uréthanes qui peuvent être utilisés en tant que groupements protecteurs, seulement quelques uns de ces uréthanes sont largement utilisés dans la synthèse peptidique. On peut notamment citer le t-butyloxycarbonyle (Boc), le benzyloxycarbonyle (Cbz) et le 9-fluorénylméthyloxycarbonyle (Fmoc). En conséquence, les N-carboxyanhydrides d'alpha-aminoacides protégés par ces substituants sont particulièrement intéressants.

Le procédé selon la présente invention permet d'éviter l'utilisation d'une amine tertiaire, en quantité très importante de 50 à 200 % molaire par rapport à la quantité molaire de NCA engagée. Les amines tertiaires utilisées dans l'art antérieur, la N-méthylmorpholine et la pyridine, posent en effet de nombreux problèmes. Notamment, leur utilisation implique un travail en haute dilution, la formation de produits parasites, une étape supplémentaire de séparation, un recyclage difficile et, de plus, elle est très coûteuse.

Le procédé selon l'invention permet également de réduire de façon notable les durées de réactions qui sont désormais inférieures à 24 heures et avantageusement de l'ordre de 1 à 4 heures, alors que pour les procédés de l'art antérieur les durées de réaction variaient de 30 heures à 5 jours.

Le procédé selon l'invention permet l'obtention de UNCAs, dont la pureté mesurée par GPC (chromatographie en phase gazeuse) est supérieure à 90%, avantageusement supérieure à 95%, avec un rendement très satisfaisant, supérieur à 60% en masse.

Selon une variante avantageuse de l'invention, le solvant est choisi dans le groupe constitué des éthers, cycliques ou linéaires, en C₄-C₁₀ et des alcanes en C₁-C₅ chlorés. Avantageusement, le solvant est le THF (tétrahydrofurane).

Lorsque le solvant de la réaction est le THF, la quantité de solvant introduite est généralement comprise entre 500 g et 2 kg de solvant pour une mole de NCA de formule II engagée.

Selon une variante avantageuse du procédé selon la présente invention, la quantité introduite de TEDA varie de 0,1 à 5% molaire de TEDA, par rapport à la quantité molaire engagée de NCA de formule II. Encore plus avantageusement, la quantité introduite de TEDA varie de 0,2 % à 1 % molaire, par rapport à la quantité molaire engagée de NCA de formule II.

Le NCA de formule II est avantageusement mis à réagir avec 1,1 à 1,5 équivalent de dicarbonate de formule III en présence de TEDA, en particulier en présence de 0,2 à 1 % molaire de TEDA par rapport à la quantité molaire engagée de NCA de formule II.

Le dicarbonate de formule III est avantageusement introduit, sous forme de solution dans une partie du solvant, avantageusement dans 0,5 et 2,0 parts en poids par rapport à la quantité totale en poids de dicarbonate engagée, régulièrement dans le milieu réactionnel comprenant l'autre partie du solvant nécessaire, le NCA de formule II à transformer et le TEDA. Pendant l'introduction du dicarbonate la température du milieu réactionnel est maintenue entre -20 et 5°C, avantageusement entre -15 et 5°C, encore plus avantageusement entre -10 et 0°C. Selon une variante avantageuse de l'invention, la réaction a lieu sous atmosphère inerte.

L'effet catalytique du TEDA autorise une technique d'introduction progressive du dicarbonate dans le milieu réactionnel, ce qui permet le contrôle de l'exothermicité par l'arrêt de l'introduction, évitant ainsi tout risque d'emballement réactionnel dangereux.

Le procédé permet de travailler en milieu beaucoup plus concentré ce qui, couplé à des durées de réactions réduites, permet un gain de productivité substantiel et limite beaucoup les risques de polymérisation.

A la fin de l'addition du dicarbonate de formule III, le milieu réactionnel est avantageusement laissé sous agitation, au moins 30 minutes à une température comprise entre -5 et 10°C.

A la fin de l'addition du dicarbonate de formule III, une fois que le milieu réactionnel a éventuellement été laissé sous agitation, le milieu réactionnel est filtré, puis au moins 80 %, avantageusement environ 90 %, du solvant est éliminé par évaporation sous pression réduite. Ensuite, un composé non-solvant est ajouté de préférence en quantité équivalente à la quantité de solvant de réaction éliminée par évaporation pour faire précipiter le UNCA de formule I qui est ensuite récupéré par filtration, le cas échéant après élimination desdits groupes protecteurs adaptés.

Selon une variante de l'invention, le solvant est éliminé par évaporation sous pression réduite à une température comprise en 15 et 30°C, avantageusement à température ambiante.

Le composé non-solvant du UNCA est avantageusement un alcane linéaire ou ramifié en C₅-C₁₀, notamment l'heptane B.

Selon une variante de l'invention, le précipité est ensuite séché sous vide à une température inférieure à 30°C.

Les exemples suivants illustrent la présente invention et ne sont pas limitatifs.

### Exemple 1 : préparation du Boc-Val-NCA :

L'abréviation Val représente l'alpha-aminoacide valine. Val-NCA représente donc le composé de formule suivante :

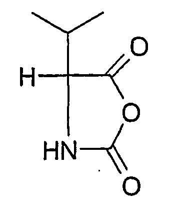

Dans un réacteur double enveloppe de 1 litre équipé de cryothermostat, d'une ampoule de coulée, d'une circulation d'azote, d'une agitation mécanique et d'une sonde thermométrique, on introduit après inertage à l'azote et refroidissement à- 5 ± 2°C :
- 204 g de THF,
- 37,5 g (0,26 mol) de Val-NCA,
- 0,15 g (1,3 mmol) de TEDA.

On agite le milieu réactionnel ½ heure puis on introduit lentement, en 2 heures, par l'intermédiaire de l'ampoule de coulée une solution de 69 g (0,316 mol) de (Boc)₂O dans 50 g de THF en régulant la température à -5°C ± 2°C. Un léger dégagement gazeux apparaît.

On maintient le milieu réactionnel sous agitation durant 1 heure après la fin de la coulée de (Boc)₂O.

On filtre le milieu réactionnel à 0°C sur pré-couche inerte montée au THF et on rince le réacteur ainsi que la pré-couche inerte par 50 mL de THF.

On replace les filtrats dans le réacteur double enveloppe toujours sous azote et on distille 300 mL de THF à une température de milieu réactionnel de 18 à 26°C sous une pression de 140 à 160 mbar.

On ajoute, à une température de 25°C, 300 mL (215 g) d'heptane B. Le produit UNCA de valine précipite. On distille alors 300 mL de mélange THF / heptane B à 25°C sous 900 - 100 mbar jusqu'à un volume de milieu réactionnel d'environ 100 mL. On ajoute ensuite 200 mL d'heptane B à une température d'environ 25°C. On refroidit le milieu réactionnel à -10°C, température que l'on maintient pendant 1 heure. On filtre sur fritté n°3, sous atmosphère d'azote à -10°C. On sèche le produit en étuve sous vide à une température de 25 ± 5°C.

On obtient ainsi 51,6 g (rendement 80,8 %) d'une poudre blanche de pouvoir rotatoire 59,1 ° (C = 1, THF) dont la pureté mesurée par GPC est de 100 %.

### Exemple 2 : préparation du Boc-Ile-NCA :

L'abréviation Ile représente l'alpha-aminoacide isoleucine. Ile-NCA représente donc le composé de formule suivante :

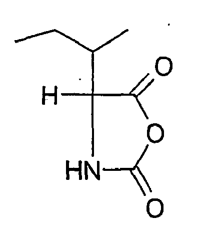

On opère comme à l'exemple 1 avec :
- 200,9 g de THF,
- 40,0 g (0,255 mol) de Ile-NCA,
- 0,14 g (1,3 mmol) de TEDA,
et une solution de 66 g (0,302 mol) de (Boc)₂O dans 66 g de THF.

Après filtration et séchage, on récupère 51,5 g (rendement 78,6 %) du produit attendu de point de fusion 107,6°C et dont le pouvoir rotatoire est de 60,3° (C = 1, THF). La pureté mesurée par GPC est de 99,3 %.

### Exemple 3 : préparation du Boc-D-Phe-NCA :

L'abréviation Phe représente l'alpha-aminoacide phénylalanine. Phe-NCA représente donc le composé de formule suivante :

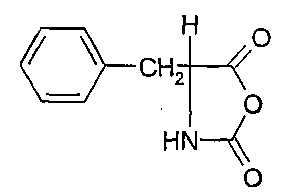

On opère comme à l'exemple 1 avec la différence que la température est fixée à -17 ± 1°C avec :
- 427 g de THF,
- 25 g (0,131 mol) de D-Phe-NCA,
- 0,07 g (0,65 mmol) de TEDA,
et une solution de 34,2 g (0,157 mol) de (Boc)₂O dans 21,5 g de THF.

Après filtration et séchage, on récupère 24,1 g (rendement 63 %) de produit conforme à la structure attendue en RMN¹H et dont la pureté mesurée par GPC est de 95,2 %.

### Exemple 4: Préparation de N-Ethoxycarbonyl-valine-N-carboxyanhydride (EtOC-Val-NCA)

Dans un réacteur double enveloppe de 1 L équipé d'un cryothermostat, d'une ampoule de coulée, d'une circulation d'azote, d'une agitation mécanique et d'une sonde thermométrique, on introduit après inertage à l'azote et refroidissement à -5 ± 2°C :
- 204 g de THF,
- 20,0 g (0,14 mol) de Val-NCA,
- 0,078 g (0,7 mmol) de TEDA..

On agite le milieu réactionnel 30 minutes puis on introduit lentement, en 1 heure, par l'intermédiaire de l'ampoule de coulée 27,1 g. (0,167 mol) de diéthyle dicarbonate [(EtOC)₂O] en régulant la température à -5°C ± 2°C. Un léger dégagement gazeux apparaît.

On maintient le milieu réactionnel sous agitation durant 1 heure après la fin de la coulée de (EtOC)₂ O.

On concentre le THF à une température de milieu réactionnel de 18 à 26°C sous une pression de 140 à 160 mbar.

On ajoute, à une température de 25°C, 220 mL d'heptane B et on verse dans une grande quantité d'eau glacée. Le produit précipite. On filtre sur fritté n°3 sous atmosphère d'azote. On sèche le produit en étuve sous vide à une température de 25 ± 5°C.

On obtient ainsi 20,1 g (rendement 67 %) d'une poudre blanche dont les spectres RMN¹H et RMN¹³C sont conformes à la structure attendue.

## Revendications

1. Procédé d'obtention de N-carboxyanhydrides d'alpha-aminoacides à protection uréthane (UNCAs), de formule I 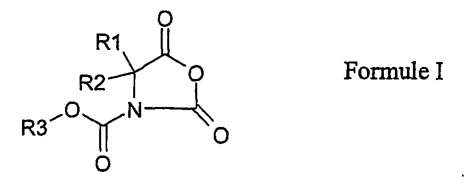 dans laquelle R1 et R2, identiques ou différents, représentent ensemble ou indépendamment l'un de l'autre un atome d'hydrogène ou une chaîne latérale d'un alpha-aminoacide naturel ou synthétique éventuellement porteuse de groupements fonctionnels le cas échéant protégés ; R³ représente un radical alkyle linéaire ou ramifié saturé ou insaturé en C₁-C₁₀ ou un radical aralkyle ou alkyl aryle de 7 à 14 atomes de carbone, **caractérisé en ce qu'**un N-carboxyanhydride d'alpha-aminoacide (NCA) de formule II, 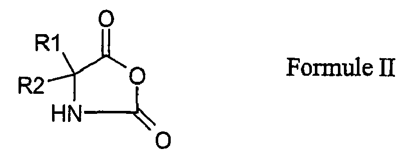 dans laquelle R1 et R2 ont la même signification que pour la formule I, est mis à réagir avec au moins un équivalent, par rapport à la quantité molaire engagée de NCA de formule II, de dicarbonate de formule III 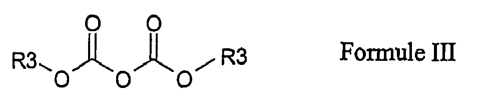 dans laquelle R3 a la même signification que pour la formule I, en présence d'une quantité catalytique de 1,4-diazabicyclo[2.2.2]octane, également dénommé triéthylène-diamine (TEDA), par rapport à la quantité molaire engagée de NCA de formule II, dans un solvant organique de point de fusion inférieur à environ -20 °C.

2. Procédé selon la revendication 1, **caractérisé .en ce que** R1 et R2, identiques ou différents, représentent un atome d'hydrogène, un radical alkyle en C₁-C₈ linéaire ou ramifié éventuellement substitué, un radical cycloalkyle en C₅-C₇ éventuellement substitué, un radical phényle, naphtyle, hétéroaromatique à 5 ou 6 chaînons ou indole éventuellement substitué, à la condition que R1 et R2 ne représentent pas chacun simultanément un radical cyclique ; ou R1 et R2 forment ensemble un radical cycloalkyle en C₅-C₇ éventuellement substitué, les substituants éventuels étant des substituants habituels dans le domaine des aminoacides et des peptides.

3. Procédé selon la revendication 2, **caractérisé en ce que** les substituants éventuels sont choisi(s) dans le groupe constitué par OH, SH, NH₂, NHC(NH)NH₂, CONH₂, O-alkyle en C₁-C₆, O-aryle en C₆-C₁₀, S-alkyle en C₁-C₆, COO-alkyle en C₁-C₆, COO-aralkyle en C₅-C₈.

4. Procédé selon l'une quelconque des revendications précédentes, **caractérisé en ce que** R3 représente le radical méthyle, le radical éthyle, le radical tertio-butyle, le radical benzyle, le radical allyle ou le radical 9-fluorénylméthyle.

5. Procédé selon l'une quelconque des revendications précédentes, **caractérisé en ce que** le solvant est choisi dans le groupe des éthers en C₄-C₁₀ et des alcanes en C₁-C₅ chlorés.

6. Procédé selon la revendication 5, **caractérisé en ce que** le solvant est le THF.

7. Procédé selon l'une quelconque des revendications précédentes, **caractérisé en ce que** la quantité de TEDA varie de 0,1 % à 5 % molaire, par rapport à la quantité molaire engagée de NCA de formule II.

8. Procédé selon la revendication 7, **caractérisé en ce que** la quantité de TEDA varie de 0,2 % à 1 % molaire, par rapport à la quantité molaire engagée de NCA de formule II.

9. Procédé selon l'une quelconque des revendications précédentes, **caractérisé en ce que** le NCA de formule II est mis à réagir avec 1,1 à 1,5 équivalent de dicarbonate de formule III, par rapport à la quantité molaire engagée de NCA de formule II.

10. Procédé selon l'une quelconque des revendications précédentes, **caractérisé en ce que** le dicarbonate de formule III est introduit sous forme de solution dans 0,5 et 2 parties en poids de solvant, par rapport à la quantité en poids de dicarbonate engagée, régulièrement dans le milieu réactionnel comprenant l'autre partie du solvant nécessaire, le NCA de formule II à transformer et le TEDA.

11. Procédé selon la revendication 10, **caractérisé en ce que** la température du milieu réactionnel est maintenue, pendant l'introduction du dicarbonate, entre -20 et 5°C, avantageusement entre -10 et 0°C.

12. Procédé selon la revendication 10 ou 11, **caractérisé en ce qu'**il comprend ensuite les étapes successives suivantes :
i) filtration du milieu réactionnel ;
ii) élimination d'au moins 80 %, avantageusement environ 90 %, du solvant par évaporation sous pression réduite ; puis
iii) ajout d'un composé non-solvant, en quantité équivalente à la quantité de solvant de réaction éliminée par évaporation, pour faire précipiter le UNCA de formule I; et
iv) récupération du UNCA de formule I par filtration, le cas échéant après élimination desdits groupes protecteurs.

13. Procédé selon la revendication 12, **caractérisé en ce que** le solvant est éliminé par évaporation sous pression réduite à une température comprise en 15 et 30°C.

14. Procédé selon l'une quelconque des revendications 12 ou 13, **caractérisé en ce que** le composé non-solvant est un alcane linéaire ou ramifié en C₅-C₁₀.

15. Procédé selon l'une quelconque des revendications 12 à 14, **caractérisé en ce que** le précipité est ensuite séché sous vide à une température inférieure à 30°C.

## Claims

1. Method for obtaining urethane-protected N-carboxyanhydrides of alpha amino acids (UNCAs), of formula I 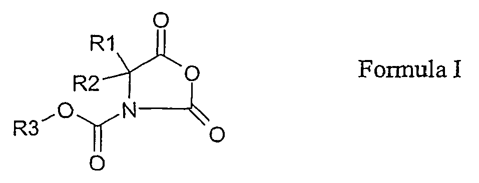 wherein R¹ and R², either identical or different, represent together or independent of each other a hydrogen atom or a side-chain of a natural or synthetic alpha-amino acid optionally bearing functional groups if necessary protected; R³ represents a saturated or insaturated, linear or branched C₁-C₁₀ alkyl radical, or an aralkyl or alkylaryl radical with 7 to 14 carbon atoms, **characterized in that** a N-carboxyanhydride of alpha-amino acid (NCA) of formula II, 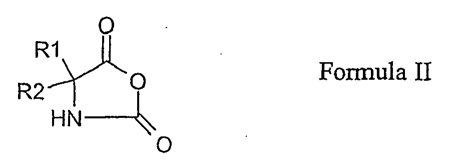 wherein R¹ and R² have the same meaning as for formula I, is reacted with at least one equivalent, relatively to the engaged molar amount of NCA of formula II, of a dicarbonate of formula III 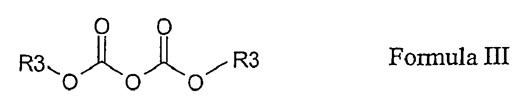 wherein R³ has the same meaning as for formula I, in the presence of a catalytic amount of 1,4-diazabicyclo[2.2.2]octane, also designated as triethylene diamine (TEDA), relatively to the engaged molar amount of NCA of formula II, in an organic solvent with a melting point less than about -20°C.

2. The method according to claim 1, **characterized in that** R¹ and R², either identical or different, represent a hydrogen atom, a C₁-C₈ linear or branched alkyl radical, optionally substituted, a C₅-C₇ cycloalkyl radical, optionally substituted, a phenyl, napthyl, 5- or 6-membered heteroaromatic radical, or indole, optionally substituted, with the proviso that R¹ and R² do not each represent a cyclic radical simultaneously; or R¹ or R² form together a C₅-C₇ cycloalkyl radical optionally substituted, the optional substituents being customary substituents in the field of amino acids and peptides.

3. The method according to claim 2, **characterized in that** the optional substituents are selected from the group consisting of OH, SH, NH₂, NHC(NH)NH₂, CONH₂, O- (C₁-C₆ alkyl), O- (C₁-C₆ aryl), S-(C₁-C₆ alkyl), COO- (C₁-C₆ alkyl), COO-(C₅-C₈ aralkyl).

4. The method according to any of the preceding claims, **characterized in that** R³ represents methyl, ethyl, tertiobutyl, benzyl, allyl, or 9-fluorenyl-methyl.

5. The method according to any of the preceding claims, **characterized in that** the solvent is selected from the group of C₄-C₁₀ ethers and chlorinated C₁-C₅ alkanes.

6. The method according to claim 5, **characterized in that** the solvent is THF.

7. The method according to any of the preceding claims, **characterized in that** the amount of TEDA varies from 0.1% to 5% molar, relatively to the engaged molar amount of NCA of formula II.

8. The method according to claim 7, **characterized in that** the amount of TEDA varies from 0.2% to 1% molar, relatively to the engaged molar amount of NCA of formula II.

9. The method according to any of the preceding claims, **characterized in that** the NCA of formula II is reacted with 1.1 to 1.5 equivalents of dicarbonate of formula III, relatively to the engaged molar amount of NCA of formula II.

10. The method according to any of the preceding claims, **characterized in that** the dicarbonate of formula III is introduced as a solution into 0.5 and 2 parts by weight of solvent, relatively to the engaged weight amount of dicarbonate, regularly in the reaction medium comprising the other required portion of solvent, the NCA of formula II to be transformed and TEDA.

11. The method according to claim 10, **characterized in that** the temperature of the reaction medium is maintained, during introduction of the dicarbonate, between -20 and 5°C, advantageously between -10 and 0°C.

12. The method according to claim 10 or 11, **characterized in that** it then comprises the following successive steps:
i) filtering the reaction medium;
ii) removing at least 80%, advantageously about 90%, of the solvent by evaporation under reduced pressure; and then
iii) adding a non-solvent compound, in an amount equivalent to the amount of reaction solvent removed by evaporation, in order to precipitate the UNCA of formula I; and
iv) recovering the UNCA of formula I by filtration, after removing said protective groups if necessary.

13. The method according to claim 12, **characterized in that** the solvent is removed by evaporation under reduced pressure at a temperature between 15 and 30°C.

14. The method according to any of claims 12 or 13, **characterized in that** the non-solvent compound is a linear or branched C₅-C₁₀ alkane.

15. The method according to any of claims 12 or 14, **characterized in that** the precipitate is then dried in vacuo at a temperature less than 30°C.

## Patentansprüche

1. Verfahren zum Erhalt von N-Carboxyanhydriden von Urethan-geschützten alpha-Aminosäuren (UNCAs) der Formel I 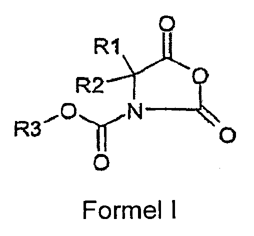 in der R1 und R2, identisch oder verschieden, zusammen oder unabhängig voneinander ein Wasserstoffatom oder eine Seitenkette einer natürlichen oder synthetischen alpha-Aminosäure darstellen, welche gegebenenfalls funktionelle Gruppen trägt, die gegebenenfalls geschützt sind; R3 einen linearen oder verzweigten, gesättigten oder ungesättigten (C₁-C₁₀)-Alkylrest oder einen Aralkyl- oder Alkylarylrest mit 7 bis 14 Kohlenstoffatomen darstellt, **dadurch gekennzeichnet, dass** ein alpha-Aminosäure-N-carboxyanhydrid (NCA) der Formel II 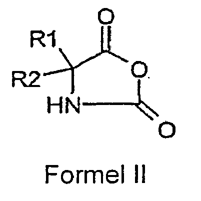 in der R1 und R2 die gleiche Bedeutung wie in Formel 1 aufweisen, mit mindestens einem Äquivalent, bezogen auf die eingesetzte Molmenge an NCA der Formel 11, Dicarbonat der Formel 111 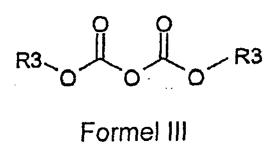 in der R3 die gleiche Bedeutung wie in Formel I aufweist, in Anwesenheit einer katalytischen Menge von 1,4-Diazabicyclo[2.2.2]octan, auch als Triethylendiamin (TEDA) bezeichnet, bezogen auf die eingesetzte Molmenge an NCA der Formel II, in einem organischen Lösungsmittel mit einem Schmelzpunkt unter etwa -20°C umgesetzt wird.

2. Verfahren nach Anspruch 1, **dadurch gekennzeichnet, dass** R1 und R2, identisch oder verschieden, ein Wasserstoffatom, einen linearen oder verzweigten, gegebenenfalls substituierten (C₁-C₈)-Alkylrest, einen gegebenenfalls substituierten (C₅-C₇)-Cycloalkylrest, einen gegebenenfalls substituierten Phenyl-, Naphthyl-, heteroaromatischen Rest mit 5 oder 6 Ringgliedern oder gegebenenfalls substituiertes Indol darstellt, mit der Maßgabe, dass R1 und R2 nicht beide gleichzeitig einen cyclischen Rest darstellen; oder R1 und R2 zusammen einen gegebenenfalls substituierten (C₅-C₇)-Cycloalkylrest bilden, wobei die fakultativen Substituenten auf dem Gebiet der Aminosäuren und der Peptide übliche Substituenten sind.

3. Verfahren nach Anspruch 2, **dadurch gekennzeichnet, dass** die fakultativen Substituenten ausgewählt sind aus der Gruppe bestehend aus OH, SH, NH₂, NHC(NH)NH₂, CONH₂, O-(C₁-C₆)-Alkyl, O-(C₆-C₁₀)-Aryl, S-(C₁-C₆)-Alkyl, COO-(C₁-C₆)-Alkyl, COO-(C₅-C₈)-Aralkyl.

4. Verfahren nach irgendeinem der vorangehenden Ansprüche, **dadurch gekennzeichnet, dass** R3 einen Methylrest, einen Ethylrest, einen Tertiärbutylrest, einen Benzylrest, einen Allylrest oder einen 9-Fluorenylmethylrest darstellt.

5. Verfahren nach irgendeinem der vorangehenden Ansprüche, **dadurch gekennzeichnet, dass** das Lösungsmittel ausgewählt ist aus der Gruppe von (C₄-C₁₀)-Ethern und chlorierten (C₁-C₅)-Alkanen.

6. Verfahren nach Anspruch 5, **dadurch gekennzeichnet, dass** das Lösungsmittel THF ist.

7. Verfahren nach irgendeinem der vorangehenden Ansprüche, **dadurch gekennzeichnet, dass** die Menge an TEDA zwischen 0,1 und 5 Mol-% variiert, bezogen auf die eingesetzte Molmenge an NCA der Formel II.

8. Verfahren nach Anspruch 7, **dadurch gekennzeichnet, dass** die Menge an TEDA zwischen 0,2 und 1 Mol-% variiert, bezogen auf die eingesetzte Menge an NCA der Formel II.

9. Verfahren nach irgendeinem der vorangehenden Ansprüche, **dadurch gekennzeichnet, dass** das NCA der Formel II mit 1,1 bis 1,5 Äquivalenten Dicarbonat der Formel III, bezogen auf die eingesetzte Molmenge an NCA der Formel II, umgesetzt wird.

10. Verfahren nach irgendeinem der vorangehenden Ansprüche, **dadurch gekennzeichnet, dass** das Dicarbonat der Formel III gleichmäßig in Form einer Lösung in 0,5 und 2 Gewichtsteilen Lösungsmittel, bezogen auf die Gewichtsmenge an eingesetztem Dicarbonat, in das Reaktionsmedium eingeführt wird, welches den anderen Teil des erforderlichen Lösungsmittels, das umzuwandelnde NCA der Formel II und das TEDA enthält.

11. Verfahren nach Anspruch 10, **dadurch gekennzeichnet, dass** die Temperatur des Reaktionsmediums während der Einführung des Dicarbonats zwischen -20 und 5°C, vorteilhaft zwischen -10 und 0°C, gehalten wird.

12. Verfahren nach Anspruch 10 oder 11, **dadurch gekennzeichnet, dass** es anschließend die folgenden aufeinanderfolgenden Schritte umfasst:
i) Filtration des Reaktionsmediums;
ii) Entfernung von mindestens 80 %, vorteilhaft etwa 90 % des Lösungsmittels durch Verdampfen unter verringertem Druck; dann
iii) Zugabe einer Nicht-Lösungsmittel-Verbindung in gleicher Menge wie die durch Verdampfen entfernte Menge des Reaktionslösungsmittels, um die UNCA der Formel 1 auszufällen; und
iv) Gewinnen der UNCA der Formel 1 durch Filtration, gegebenenfalls nach Entfernen der Schutzgruppen.

13. Verfahren nach Anspruch 12, **dadurch gekennzeichnet, dass** das Lösungsmittel durch Verdampfen unter verringertem Druck bei einer Temperatur zwischen 15 und 30°C einschließlich entfernt wird.

14. Verfahren nach irgendeinem der Ansprüche 12 oder 13, **dadurch gekennzeichnet, dass** die Nicht-Lösungsmittel-Verbindung ein lineares oder verzweigtes (C₅-C₁₀)-Alkan ist.

15. Verfahren nach irgendeinem der Ansprüche 12 bis 14, **dadurch gekennzeichnet, dass** der Niederschlag anschließend unter Vakuum bei einer Temperatur unter 30°C getrocknet wird.
